# FASCICULE DE BREVET EUROPEEN

(11) **EP 1 513 506 B1**
(45) Date de publication et mention de la délivrance du brevet: **19.10.2011**
(21) Numéro de dépôt: 03760766.0
(22) Date de dépôt: 20.06.2003
(51) Int. Cl.: A61K 9/51

(54) **SYSTEME DE VECTORISATION COMPRENANT DES NANOPARTICULES DE TAILLE HOMOGENE D'AU MOINS UN POLYMERE ET D'AU MOINS UN POLYSACCHARIDE CHARGE POSITIVEMENT ET SON PROCEDE DE PREPARATION**
VEKTORSYSTEM ENTHALTEND NANOPARTIKELN GLEICHMÄSSIGER GRÖSSE,WELCHE AUS ZUMINDESTENS EINEM POLYMER UND EINEM POSITIV GELADENEN POLYSACCHARID BESTEHEN,UND HERSTELLUNGSVERFAHREN DIESES SYSTEMS
TARGETING SYSTEM COMPRISING UNIFORMLY-SIZED NANOPARTICLES WITH AT LEAST ONE POLYMER AND AT LEAST ONE POSITIVELY-CHARGED POLYSACCHARIDE AND PREPARATION METHOD THEREOF

(30) Priorité: 20.06.2002 FR 0207668
(43) Date de publication de la demande: 16.03.2005
(73) Titulaire: BioAlliance Pharma, 75015 Paris (FR)
(72) Inventeur: ABOUBAKAR, Malam, F-59000 Lille (FR); MAKSIMENKO, Andrei, F-91800 Brunoy (FR); CHAUMONT, Christine, F-31400 Toulouse (FR); POLARD, Valérie, F-94140 Alfortville (FR)
(74) Mandataire: Audic, Hervé
(86) Numéro de dépôt international: PCT/FR2003/001909
(87) Numéro de publication internationale: WO 2004/000287

(56) Documents cités:
- WO-A-96/24377
- WO-A-99/43359
- FR-A- 2 649 321
- FR-A- 2 724 935
- US-A- 4 913 908

## Description

L'invention concerne la délivrance de principes actifs utilisés notamment dans le domaine des médicaments à visée préventive, curative ou diagnostique. Elle a plus particulièrement pour objet un système de délivrance et de vectorisation de substances d'intérêt constitué de nanoparticules à base d'au moins un polymère, de préférence un poly(cyanoacrylate d'alkyle) et d'au moins un polysaccharide chargé positivement, de préférence un chitosan de bas poids moléculaire et des substances d'intérêt anioniques, ou comprenant une région anionique, adhérées ou incorporées au niveau desdites nanoparticules.

La mise au point de nouveaux systèmes de délivrance ou libération de principes actifs a pour objectif premier la délivrance contrôlée d'un agent actif, notamment pharmacologique, à son site d'action à une vitesse et à une posologie thérapeutiquement optimale (J. Kreuter, 1994). On peut aussi citer l'amélioration de l'index thérapeutique, qui peut être obtenue par modulation de la distribution du principe actif dans l'organisme. L'association du principe actif au système de délivrance permet notamment sa délivrance spécifiquement au site d'action ou sa libération contrôlée après le ciblage du site d'action. La réduction de la quantité de principe actif dans les compartiments où sa présence n'est pas souhaitable permet d'accroître l'efficacité dudit principe actif, de réduire ses effets secondaires toxiques, voire même de modifier ou de restaurer son activité.

Une des questions majeure de la délivrance est son application à la biologie moléculaire et tout particulièrement l'application à des principes actifs comme les acides désoxyribonucléiques (ADN), les oligodésoxynucléotides (ODN), les peptides, les protéines chargés tous négativement. La compréhension des bases de biologie moléculaire dans les maladies génétiques permet de moduler ou de remplacer un gène en disfonctionnement. Le développement de la thérapie génique en routine dans la pratique clinique dépend de la possibilité d'administration par voie systémique de façon répétée, de la capacité à atteindre une cible et à transfecter efficacement des cellules *in vivo* (Felgner, 1990, Kabanov et Alakhov, 1993, Crook, 1995, Douglas et Curiel, 1995, Lasic et Templeton, 1996) ainsi que de la possibilité de fabriquer des vecteurs qui seront capables d'être transposés à l'échelle industrielle. Les techniques de transfection *in vitro* utilisées jusqu'à présent, comme l'électroporation et la co-précipitation de l'ADN avec le phosphate de calcium sont des approches dont l'application *in vivo* paraît difficile (Fynan *et al.,* 1993).

Les systèmes actuellement utilisés pour la transfection *in vivo* tiennent compte de la charge naturelle négative des ADN ou des ODN. Ce sont soit des systèmes viraux (Morgan et Anderson, 1993) soit des constructions non virales comme des lipides cationiques (Ledley, 1994, Zelphati et Szoka, 1996). Les vecteurs viraux sont très efficaces en termes de transfection *in vitro,* mais ils possèdent des limitations *in vivo* à cause de leur immunogénécité (Wilson et al., 1990, Douglas et Curiel, 1995 ; Verma et Somia, 1997). Parmi les vecteurs non viraux, les lipides cationiques tels que le Transfectam (Behr et al., 1989) présentent de bonnes propriétés de transfection, mais leur application *in vivo* est limitée par leur toxicité, l'activation du complément et leur important tropisme pour le foie et les poumons.

Depuis les études effectuées avec la poly(1-lysine) à la fin des années 1980 (Wu et Wu, 1987), plusieurs polymères cationiques ont été étudiés comme vecteurs de transfection non viraux. Ces vecteurs incluent la polyéthylène-imine (Boussif *et al.,* 1995 ; Remy *et al.,* 1998), le polybrene (Mumper *et al.,* 1996), les dendrimers de poly(amidoamine) (PAMAM) (Tang *et al*., 1996).

Jusqu'à présent, l'efficacité de transfection avec les polymères est relativement faible et beaucoup de ces polymères cationiques ont montré une toxicité relative avec de faibles potentiels d'administration répétée par voie intraveineuse. Les polymères utilisés directement avec les principes actifs notamment l'ADN sont limités par leur efficacité de transfection et consomment une grande quantité de principe actif et de ce fait obligent à utiliser une grande quantité de polymère, elle même toxique. Les systèmes colloïdaux utilisés pour la délivrance notamment de gènes *in vivo* sont plus économes en polymères et en principe actif.

Les systèmes colloïdaux de délivrance de principes actifs comprennent les liposomes, les microémulsions, les nanocapsules, les nanosphères, les microparticules et les nanoparticules. Les nanoparticules présentent des avantages de ciblage, de modulation de distribution et de souplesse de formulation et ont une structure polymère qui peut être conçue et réalisée de façon adaptée au but poursuivi. Elles se sont révélées tout particulièrement prometteuses pour obtenir un meilleur index thérapeutique au sens défini ci-dessus, en raison de leur aptitude à assurer une libération contrôlée, une délivrance spécifique au site d'action ou délivrance ciblée, permettant à la fois une augmentation de l'efficacité et une réduction des effets secondaires toxiques au niveau des autres organes.

Parmi ceux-ci, les poly(cyanoacrylates d'alkyle) décrits dans les EP-B-0 007 895 (US-A-4,329,332 & US-A-4,489,055),
FR 2 649 321-A, FR 2 724 935-A et EP-B-0 064 967 (US-A-4,913,908) sont particulièrement intéressants car leur bioérosion est observée rapidement par rapport à d'autres polymères biodégradables et se déroule pendant des durées compatibles avec les applications thérapeutiques ou diagnostiques. Les nanoparticules sont des vecteurs colloïdaux dont le diamètre varie entre 10 nm et 1000 nm. Ces particules sont formées par des macromolécules dans lesquelles la substance biologiquement active est piégée, encapsulée ou adsorbée à la surface. Les nanoparticules ou nanosphères ont été décrites par Birrenbach et Speiser (1976) en terme de « nanopellet », et nanocapsules, et qualifiées par Kreuter et Speiser (1976) d'« adjuvants » et de « systèmes de délivrance de substances actives » par Kreuter (1983).

Dans le but d'augmenter la stabilité des oligonucléotides, d'augmenter leur pénétration dans les cellules et d'éviter la distribution non spécifique, l'utilisation de vecteurs particulaires, comme les nanoparticules, est considérée comme une approche des plus prometteuses. Cependant leur utilisation a été limitée par la toxicité de la substance utilisée pour fixer le principe actif à la nanoparticule.

Les nanoparticules polymères les plus étudiées sont les polyisohexylcyanoacrylates (PIHCA). Cependant, comme ces nanoparticules présentent une charge négative en surface, un copolymère cationique (Diethylaminoethyl (DEAE) Dextran) ou un détergent hydrophobe cationique (Cétyl triméthyl ammonium bromide (CTAB)) ont été combinés aux polyalkylcyanoacrylates (PACA) afin de faciliter l'association des ODN par formation de paires d'ions sur les nanoparticules. Ainsi, les ODN ont été associés efficacement aux nanoparticules de PACA contenant un cation hydrophobe tel que le CTAB (Cétyl triméthyl ammonium bromide, Chavany *et al.*, 1992). Le CTAB présente des problèmes de toxicité, Zobel *et al*., 1987 ont remplacé le CTAB par le DEAE dextran qui a été introduit dans le milieu de polymérisation avant la formation des nanosphères. Le DEAE Dextran s'est avéré également toxique.

Le vecteur nanoparticulaire idéal pour la délivrance des principes actifs comme les ADN, les ODN, les peptides, les protéines, doit être :
- capable de former un complexe avec la molécule d'intérêt en prenant en compte ses caractéristiques physico-chimiques et la substance choisie, pour fixer le principe actif, doit être adaptée à la charge et aux données de toxicité,
- capable de protéger le principe actif de la dégradation pendant son transport dans la circulation sanguine,
- biocompatible (non toxique, non immunogène, et de préférence biodégradable),
- capable de délivrer le principe actif au niveau du tissu cible en quantité suffisante, et
- capable de cibler spécifiquement un type cellulaire.

Ce but est atteint grâce à l'utilisation de nanoparticules de polymères, en particulier de poly(cyanoacrylates d'alkyle), associés à un ou plusieurs polysaccharides chargés positivement.

L'invention a donc pour objet un système de vectorisation et de délivrance de substances d'intérêt thérapeutique ou de diagnostic, du type constitué de nanoparticules, caractérisé en ce que lesdites nanoparticules présentent une distribution de taille homogène et comprennent (i) au moins un polymère et (ii) au moins un polysaccharide chargé positivement non toxique.

Dans le système selon l'invention, les substances d'intérêts sont internalisées ou adsorbées au niveau desdites nanoparticules.

Le système de vectorisation et de délivrance selon l'invention est tout particulièrement adapté à des substances d'intérêt anioniques ou comprenant une région anionique.

De préférence le polymère est un poly(cyanoacrylate d'alkyle) dans lequel le groupe alkyle, linéaire ou ramifié, comprend de 1 à 12 atomes de carbone.

Le(s) polysaccharide(s) entrant dans la composition des nanoparticules de l'invention sont non toxiques, à la différence des substances proposées dans l'art antérieur comme le CTAB ou le DEAE-Dextran.

Ainsi, l'invention concerne tout particulièrement, un polysaccharide chargé positivement qui est linéaire, il s'agit de préférence d'un dérivé de la chitine et tout préférentiellement du chitosan ou ses dérivés dès lors qu'ils sont chargés positivement.

La Chitine est un polysaccharide abondamment trouvé dans la nature après la Cellulose. Le squelette est composé de sucres β1-4-glucosamine avec un haut degré de N-acétylation. Cette structure est proche de celle de la Cellulose, la différence étant le remplacement de la fonction hydroxyl par un groupement amino (Roberts, 1992). Ces biopolymères polycationiques constituent l'exosquelette des crustacés et des insectes, mais ils sont également présents dans certains champignons (Roberts, 1992). Le principal dérivé de la Chitine nommé Chitosan est usuellement obtenu par dé-acétylation alkaline. Les deux types de polymères (Chitine et Chitosan) se différencient par leur solubilité ou insolubilité dans une solution d'acide diluée (Roberts, 1992).

Le Chitosan de par ses propriétés biologiques favorables telles que sa non-toxicité (Knapczyk et *al*., 1984), sa biocompatibilité (Chandy et Sharma, 1990 ; Hirano et *al*., 1990) et sa biodégradabilité (Struszczyk et *al*., 1991) a beaucoup été étudié dans les domaines pharmaceutique et biomédical. Dans le domaine médical, le Chitosan a été décrit comme possédant des propriétés pharmacologiques intéressantes telles que son activité hypocholestérolémiante (Kobayashi *et al*., 1979), ses propriétés cicatrisantes (Balassa et Grove, 1972), et ses activités anti-acides et anti-ulcérantes (Hillyard et *al*., 1964). De plus, son caractère polycationique lui confère la capacité à fortement s'associer aux cellules mammaliennes, permettant des potentialités d'utilisation pour son activité hémostatique (Fradet *et al.*, 1986) et son activité spermicide (Smith, 1984).

Le poids moléculaire des polysaccharides utilisés dans l'invention a été soigneusement sélectionné afin d'obtenir des nanoparticules homogènes et de taille suffisamment petite pour faciliter leur administration. Ainsi, ces polysaccharides, et tout particulièrement le chitosan ou ses dérivés, ont une masse moléculaire inférieure à 100 000 Da, de préférence inférieur 30 000 Da. Du chitosan de 5000, 10000 et 30000 Da a été synthétisé dans le cadre de l'invention.

A titre d'exemple de dérivés de chitosan, on peut citer Chitosan peggylé, c'est-à-dire du chitosan auquel on a greffé du polyéthylène glycol ou du Chitosan fonctionnalisé avec l'acide folique pour le ciblage des cellules cancéreuses.

Le système de délivrance et de vectorisation selon l'invention permet de moduler la délivrance de la substance d'intérêt en fonction de la quantité de polysaccharide chargé positivement dans les nanoparticules. Cette modulation de charge était impossible dans l'art antérieur à cause de la toxicité des détergents comme le CTAB ou des copolymères comme le DEAE-Dextran. Ainsi dans le système de l'invention, la quantité de polysaccharide est comprise entre 0,1 et 1 % par rapport à la quantité de polymère.

Les nanoparticules de l'invention peuvent aussi comprendre au moins un composé apte à complexer la substance d'intérêt. Il s'agit par exemple d'un composé décrit dans le brevet français publié sous le No. 2 775 435. Avantageusement, le composé apte à complexer la substance active est un oligosaccharide cyclique, de préférence une cyclodextrine neutre ou chargée, native, branchée ou polymérisée ou modifiées chimiquement. Il peut s'agir d'une cyclodextrine modifiée chimiquement par substitution d'un ou plusieurs hydroxypropyles par des groupements alkyle, aryle, arylalkyle, glycosidique, ou par estérification avec des alcools ou des acides aliphatiques.

Le système de vectorisation et de délivrance selon l'invention est tout particulièrement adapté à des substances d'intérêt anioniques ou comprenant une région anionique. Ainsi, il peut aussi s'agir de substances d'intérêt qui ne sont pas chargées, ou chargées positivement mais qui sont modifiées de façon à leur conférer en totalité ou en partie une charge négative permettant leur association aux nanoparticules, selon l'invention, chargées positivement. La substance d'intérêt se fixe sur les nanoparticules de façon covalente ou non.

On peut citer par exemple l'ADN ou des oligodésoxyribonucléotides ou des oligoribonucléotides notamment des antisens ou des ARN interférent (ARNsi), ou encore des peptides, polypeptides ou protéines, des composés chimiques. Les acides nucléiques sont des substances anioniques, mais certains peptides, polypeptides ou protéines sont également anioniques ou comprennent une région chargée négativement. A titre d'exemples de substances d'intérêt susceptible d'être associées aux nanoparticules de l'invention, on peut citer l'insuline, la calcitonine, la triptoréline aux pH supérieurs à leur pHi.

Les nanoparticules selon l'invention présentent un diamètre homogène compris entre 10 et 300 nm en fonction du PM de Chitosan et de la concentration utilisée. Elle peuvent être préparées par des techniques de polymérisation en émulsion (Couvreur et al., 1979) du type décrit dans le brevet français No. 2 775 435 ainsi que dans la partie expérimentale ci-après.

L'invention a tout particulièrement pour objet un procédé de préparation de ces nanoparticules comprenant la polymérisation à pH acide supérieur à 1 des monomères en présence du polysaccharide chargé positivement.

De préférence le polysaccharide est ajouté en début de polymérisation et le pH acide est supérieur à 3. Selon une forme de réalisation toute préférée de ce procédé la polymérisation est réalisée en présence d'un composé ralentisseur de polymérisation comme SO₂ ou l'hydroquinone, qui se fixe sur les groupes OH du milieu aqueux de polymérisation ou du polysaccharide.

On obtient par le procédé ci-dessus une suspension de nanoparticules de polymère et de polysaccharide dont la charge positive dépend de la quantité de polyssacharide ajoutée. La quantité de polysaccharide est avantageusement comprise entre 0,01% et 5% et de préférence comprise entre 0,1% et 1% par rapport au poids de la suspension.

La substance d'intérêt cationique peut être ajouté à tout moment du procédé ci-dessus. En effet, la polymérisation à un pH, certes acide mais supérieur à 1, permet d'introduire dès le début du procédé des substances d'intérêt sensibles aux pH trop acide. La quantité de substance d'intérêt est avantageusement comprise entre 0,5% et 50% par rapport au poids de la suspension.

A titre d'exemple spécifique, on peut citer le procédé suivant :
100 µl de polyisohexylcyanoacrylate contenant 0 à 20000 ppm du SO₂ sont ajoutés dans une solution d'acide citrique (avec un pH variant de 1 à 5) contenant 1% de tensioactif non ionique, le poloxamer 188, et éventuellement 0,5% de cyclodextrine et contenant 0,2% de chitosan de faible poids moléculaire (5000 ou 10000 Da).

On peut rappeler que l'utilisation de chitosan pour obtenir des nanoparticules chargées positivement a déjà été décrite dans l'art antérieur (Yang S.C. et al., 2000). Cependant le procédé de l'art antérieur est réalisé à un pH du milieu de polymérisation très acide pH (pH1) auquel les principes actifs ne sont pas stables.

Dans le procédé selon la présente invention, le pH du milieu de polymérisation est compris entre 2,5 et 5, où les principes actifs sont stables. En outre, le procédé décrit par Yang S.C. et al. met en ouvre un chitosan de poids moléculaire supérieur à 30000 Da. Or, l'utilisation de chitosan de poids moléculaire supérieur à 10000 Da présente une toxicité (Richardson S.C.W., et al., 1999). Au contraire, les nanoparticules selon la présente invention comprennent du chitosan de bas poids moléculaire (5000 et 10000 Da), ce qui permet en outre d'obtenir des nanoparticules présentant une distribution en taille monodisperse et de ce fait avec une meilleure tolérance

Le procédé décrit par Yang S.C. et al. est limité par la concentration de chitosan présente dans le milieu de polymérisation, car à une concentration de chitosan inférieure à 0,25%, les nanoparticules obtenues sont instables. Le procédé selon l'invention permet d'obtenir des nanoparticules stables pour des concentrations de chitosan inférieures à 0,25%.

Le système de vectorisation et de délivrance d'une substance d'intérêt selon l'invention peut se présenter sous la forme d'une suspension de nanoparticules, ou sous forme de lyophilisat de nanoparticules. Il permet une administration sans dénaturation de la substance d'intérêt par voie intra-veineuse, par voie orale, par voie locale.

D'autres avantages et caractéristiques de l'invention apparaîtront des exemples qui suivent concernant la préparation des nanoparticules de l'invention et leur capacité à transporter des oligonucléotides, et dans lesquels il sera fait référence aux dessins en annexe dans lesquels :
- La figure 1 représente la toxicité des différentes préparations sur les cellules NIH 3T3 *EWS-Fli*1.
- La figure 2 représente la toxicité des différentes préparations sur les cellules NIH 3T3.
- La figure 3 représente la toxicité des différentes préparations sur les cellules IW 35.
- La figure 4 représente la toxicité des différentes préparations sur des cellules CEM 4FX.
- La figure 5 représente la toxicité des NP Chitosan 5 KDa et 5-10 KDa et des NP CTAB sur des cellules CEM4FX après 24 h d'incubation.
- La figure 6 représente l'adsorption d'ODN EF3008AS sur les nanoparticules Chitosan 5 KDa et les nanoparticules CTAB.
- La figure 7 représente la stabilité de l'ON EF3008AS.
- La figure 8 montre les Nanoparticules de Chitosan 5KD avec ON 11 mer (D) et ON 7 mer OPC (B) dans les cellules CEM4FX.
- La figure 9 montre les Nanoparticules de Chitosan 5KD avec ON 11 mer Fluo dans les cellules IW 35.
- La figure 10 montre les Nanoparticules de Chitosan 5KDa (A) et 5-10 kDa (B) avec ON EFAS 30-mer
- La figure 11 représente la pénétration du plasmide marqué à la Rhodamine, lors de transfection *in vitro* des cellules NIH-3T3 EWS-Flil par des complexes plasmide-NP-Chitosan.
- La Figure 12 représente l'expression de la β-gal dans les cellules NIH3T3 EWS/Fli-1 greffées sur des souris nude après marquage immunologique avec des anti corps anti β-gal. (A) transfection avec 10 µg de plasmide pCMVβ-gal, (B) transfection avec 10 µg de plasmide pCMVβ-gal associé à 60 µg de NP-chitosan 5 kDa.
- La figure 13 représente la localisation de l'ON EF3008AS-FITC vectorisé par les NP-chitosan <5kDa (A) et chitosan 5-10 kDa (B) dans les cellules NIH 3T3 EWS/Fli-1 après observation par microscopie confocale.
- La figure 14 représente la localisation intracellulaire de l'ON EF3008AS-FITC vectorisé par des NP/chitosan <5kDa (A) et chitosan 5-10 kDa (B) après balayage d'un champ des cellules par le laser du microscope confocal.
- La figure 15 représente l'inhibition de la croissance tumorale après administration intratumorale : du PBS, NP-chitosan 5 kDa ODN EF3008AS, NP-chitosan 5 kDa ODN control et ODN EF3008AS à des souris *nude* irradiées qui ont développé une forme sous-cutanée du sarcome d'Ewing.
- La Figure 16 représente l'inhibition de la croissance tumorale après injection intraveineuse de NP-chitosan 5KDa ODN EF3008AS (ON AS/NP), ODN EF3008AS (ON AS), ODN contrôle (ON CON), NP-chitosan 5kDa ODN contrôle (ON CON/NP), NP-chitosan 5kDa (NP) et NaCl à des souris *nude* irradiées qui ont développé une forme sous-cutanée du sarcome d'Ewing.
- La Figure 17 représente la détection de l'expression de la protéine GFP dans la tumeur par la technique d'immunohistochimie : A (coupe de la tumeur), B (coupe de la tumeur après administration intra tumorale de l'ARNsi antisense), C (coupe de la tumeur après administration intra tumorale de l'ARNsi contrôle) D (coupe de la tumeur après administration intra tumorale de l'ARNsi antisense vectorisé par les NP-chitosan 5kDa) E (coupe de la tumeur après administration intra tumorale de l'ARNsi contrôle vectorisé par les NP-chitosan 5kDa).
- La Figure 18 représente la détection de l'inhibition de la synthèse de l'ARNm de la protéine GFP par la technique de Northern blot. L'ARNm GFP a été normalisé par l'ARNm 28s.

### Exemple I : Etude de la Toxicité comparée du Chitosan de différents poids moléculaires et du CTAB sur différentes lignées cellulaires.

### 1) Matériels et méthodes.

Les cellules NIH3T3 et NIH 3T3 EWS-Fli-1 (cellules adhérentes) sont ensemencées en plaques 6 puits à 100 000 cellules par puits dans leur milieu de culture sans puromycine et les cellules IW35 (cellules en suspension) en plaques 12 puits à raison de 750 000 cellules par puits. D'autres cellules en suspension CEM 4FX ont également été testées, elles sont ensemencées à 10 000 cellules par puits dans les plaques à 96 puits.

Pour les deux premiers types cellulaires, les cellules sont ensemencées 24 heures avant leur transfection. Le milieu est alors éliminé et 100 µL de solution du complexe à tester sont mis au contact des cellules dans 900 µL de milieu.

Différentes concentrations de CTAB et de Chitosan de plusieurs poids moléculaires ont été testées.

Deux témoins sont utilisés et correspondent aux cellules dans du milieu de culture d'une part et dans du milieu de culture contenant 100µL de NaCl 150 mM d'autre part. Après 24 heures d'incubation, les mesures de cytotoxicité sont effectuées par un test mesurant l'activité des enzymes déshydrogénases mitochondriales des cellules vivantes par oxydation du MTT (Bromure de 3-(4,5 diméthyl thiazol-2-yl)-2,5-diphényl tétrazolium ; SIGMA) en Formazan. La quantité de Formazan produite est directement proportionnelle au nombre de cellules vivantes métaboliquement actives. Les cellules sont lavées au PBS et 1ml du milieu est ajouté. Les cellules sont ensuite incubées à 37°C avec 100 µl d'une solution du MTT (5mg/ml dans du PBS) pendant 3 heures. 1 mL d'une solution de lyse (10% SDS, 10 mM Tris,) est ensuite ajouté dans chaque puits. Les cellules sont incubées à 37°C pour la nuit. Le milieu est homogénéisé et la densité optique est lue à 560 nm (Dynatech MRX).

### 2) Résultats.

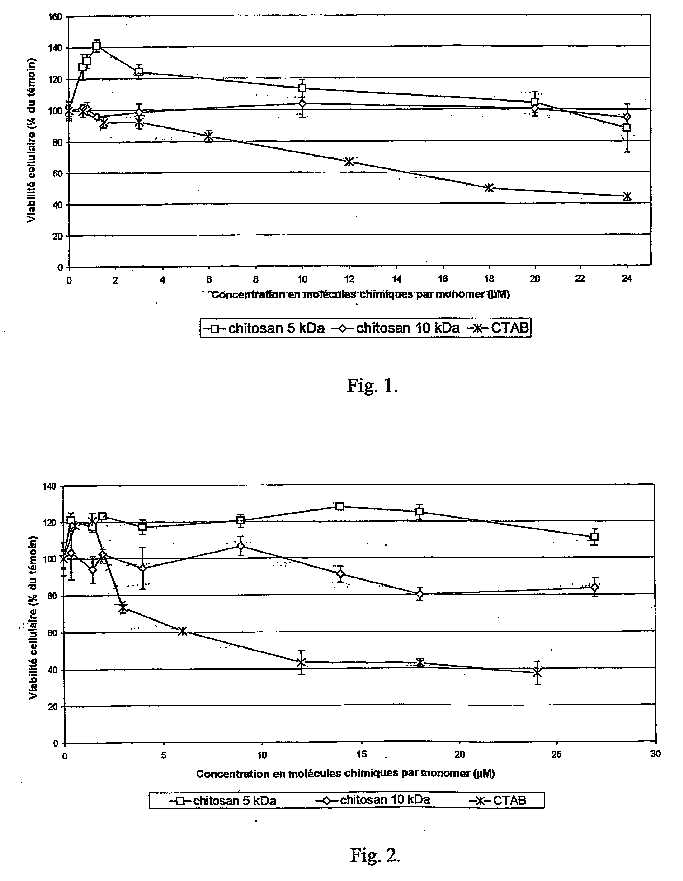

La toxicité des différentes préparations sur les différentes cellules (NIH 3T3, NIH 3T3 *EWS-Fli*1, IW 35 et CEM 4FX) est représentée en fonction de la concentration en molécule chargées positivement (figure 1-4). Pour les cellules NIH 3T3 avec et sans *EWS-Fli*1 (cellules adhérentes) (figures 1-2) on constate une importante toxicité du CTAB comparée au Chitosan de 5 ou 10 KDa. De plus le Chitosan de 5 KDa n'est pas toxique et stimule la viabilité cellulaire.

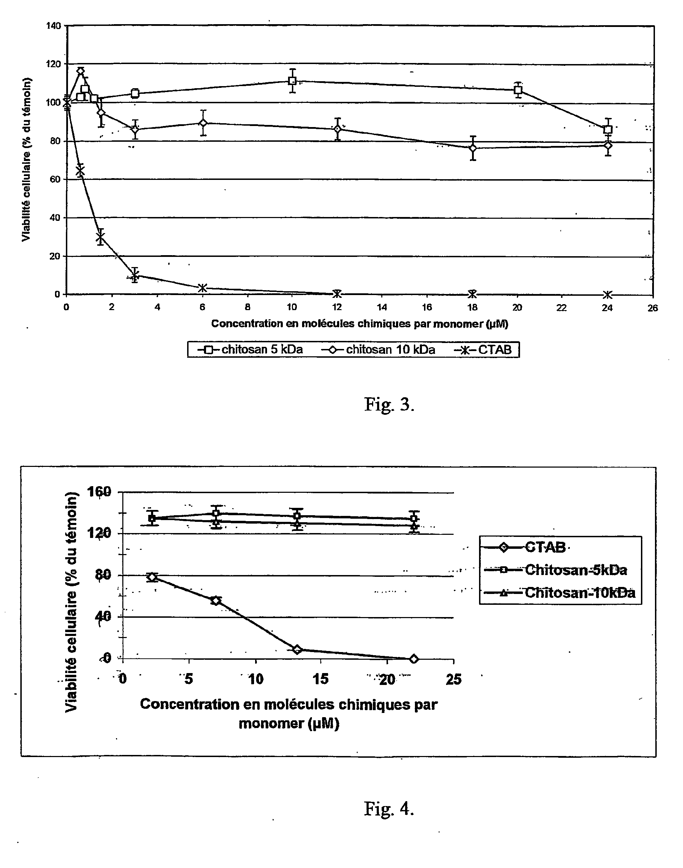

Pour les cellules en suspension (IW35 et CEM 4FX) (figure 3 et 4), les 2 types de Chitosan 5 et 10 KDa ne présentent pas de toxicité importante comparées aux CTAB qui pour de très faibles concentrations présentent des toxicités élevées.

### Exemple II : Formulation de nanoparticules chargées positivement en présence de Chitosan de différents poids moléculaires.

Les nanoparticules sont préparées par la technique de polymérisation en émulsion (Couvreur et al., 1979). 100 µL de polyisohexylcyanoacrylate contenant du SO₂ (Monorex®) sont ajoutés dans une solution d'acide citrique dilué à pH=3 contenant 1% de tensioactif non ionique, le poloxamer 188, contenant ou pas 0,5% de Cyclodextrine et contenant 0,2% chitosan de faible poids moléculaire (5 000 ou 10 000 Da). La durée de polymérisation est de 6 heures. Après polymérisation, la taille a été déterminée après dilution des particules dans de l'eau MilliQ et le potentiel zéta a été déterminé après dilution des particules dans du NaCl 1 mM. Le tableau 1 ci-dessous rapporte la taille et le potentiel zéta des nanoparticules en fonction du poids moléculaire du chitosan.

**Tableau 1**

| | Diamètre (nm) | Ecart type | Indice de polydispersité | Potentiel Zêta |
|---|---|---|---|---|
| NP/chitosan PM 5 KDa | 58 | 16 | 0,025 | +21 |
| NP/chitosan PM 5-10 KDa | 60 | 15 | 0,065 | +22 |

Le diamètre des particules ne varie pas de en fonction du PM de Chitosan. Quel que soit le PM de chitosan utilisé à la concentration de 0.2%, on obtient des nanoparticules très petites et monodisperses comme l'atteste la valeur de l'indice de polydispersité.

Le potentiel zéta de ces particules est positif et la charge est indépendante du poids moléculaire du chitosan.

### Exemple III : Etude de la toxicité comparée des nanoparticules chargées en Chitosan et en CTAB.

### 1) Matériels et méthodes.

Les cellules en suspension CEM 4FX ont été ensemencées à raison de 10 000 cellules par puits dans des plaques à 96 puits. Différentes concentrations de nanoparticules possédant des charges positives par ajout du CTAB ou du Chitosan 5 KDa et 10 KDa ont été testées.

Deux témoins sont utilisés et correspondent aux cellules dans du milieu de culture d'une part et dans du milieu de culture contenant 100 µL de NaCl 150 mM d'autre part. Après 24 heures d'incubation, les mesures de cytotoxicité sont effectuées par un test mesurant l'activité des enzymes déshydrogénases mitochondriales des cellules vivantes par oxydation du MTT en Formazan. La quantité de Formazan produite est directement proportionnelle au nombre de cellules vivantes métaboliquement actives. Les cellules sont lavées au PBS et 1ml du milieu est ajouté. Les cellules sont ensuite incubées à 37°C avec 100 µl d'une solution du MTT (5 mg/ml dans du PBS) pendant 3 heures. 1 mL d'une solution de lyse (10% SDS, 10 mM Tris,) est ensuite ajoutée dans chaque puits. Les cellules sont incubées à 37°C pour la nuit. Le milieu est homogénéisé et la densité optique est lue à 560 nm (Dynatech MRX).

### 2) Résultats.

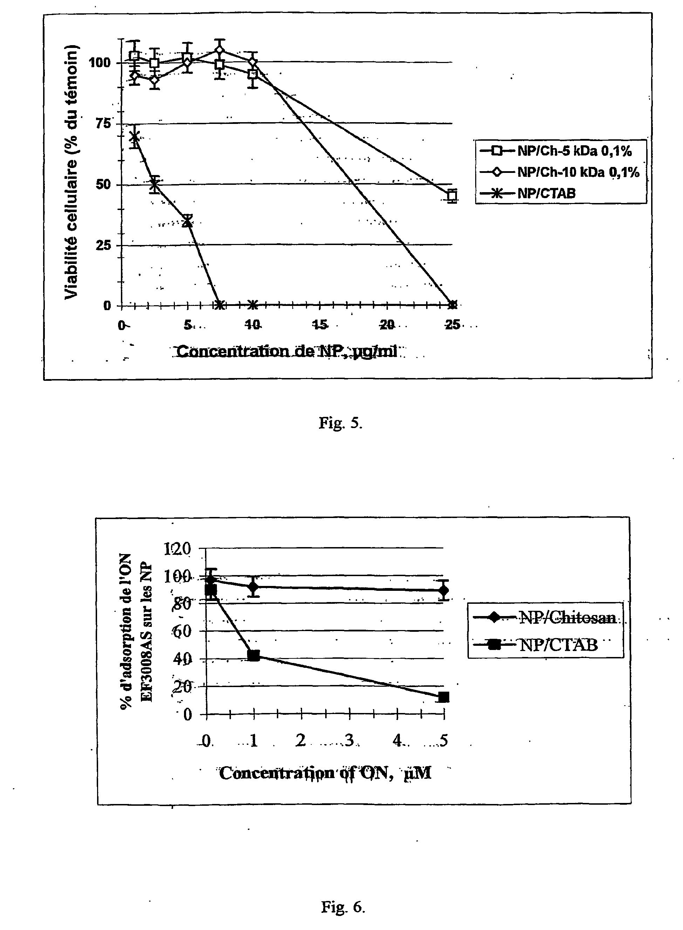

Sur la lignée de cellules CEM 4FX, les nanoparticules de Chitosan 5 et 10 KDa présentent une toxicité à partir de 10 µg/mL uniquement, alors que les nanoparticules chargées en CTAB sont toxiques à partir de 1 µg/mL (figure 5).

### Exemple IV: Formulation de nanoparticules chargées en oligonucléotides.

Les oligonucléotides sont des macromolécules chargées négativement capables d'interagir avec des surfaces chargées positivement.

Nous avons étudié la formation de complexes des NP/chitosan avec des oligonucléotides antisens EF3008AS.

Les ON anti-sens sont des séquences nucléiques capables de s'hybrider sélectivement avec des ARNs messagers cellulaires-cibles pour inhiber leur traduction en protéine. Ces ON forment avec L'ARNm-cible, de manière locale, des régions double brin, par des interactions de type Watson-Crick classique.

### 1) Matériels et Méthodes.

Les solutions mères de nanoparticules (10 mg/ml) sont diluées à la concentration finale de 1mg/mL dans du tampon Tris-HCl, pH=7 à 10 mM en concentration finale. L'adsorption des oligonucléotides sur les nanoparticules de Chitosan se fait dans 150 mM de NaCl pendant 30 minutes à température ambiante après avoir vortexé les solutions pendant environ 30 secondes. Les complexes sont ensuite incubés pendant 30 minutes à température ambiante.

La formation de complexes nanoparticules CTAB-ODN s'effectue en présence de CTAB (à raison de 3 µmoles de CTAB pour 5 µg de NP) et d'ODN dans 150 mM de NaCl. Les ODN sont adsorbés sur les NP-CTAB au bout de 2 heures sous agitation à température ambiante. La préparation des complexes se fait dans un volume final de 200 µL.

Différentes quantités d'ODN 30 mer (EF3008AS) (20, 50, 100, 125, 150, et 200 µg) sont mélangées avec 100 µg de NP-Chitosan 5 KDa, NP-Chitosan 5-10 KDa.

Les nanoparticules chargées en ODN, de même que les ODN seuls sont centrifugés à 35 000 rpm (Beckman Optima L80, rotor TW50, 1) pendant 30 minutes à 4°C. La quantité d'ODN adsorbée aux NP a été calculée en déterminant la quantité d'ODN libre dans les surnageants (figure 6).

### 2) Résultats.

Pour des ratios ON/NP de 0,2/1 et 0,5/1 on obtient une adsorption d'environ 100% d'ON antisens EF3008AS sur les nanoparticules de chitosan 5 KDa. Pour les nanoparticules de chitosan 5-10 KDa on obtient une adsorption de 100% pour le ratio ON/NP de 0,2/1, le pourcentage d'adsorption est de 80% quand on passe au ratio ON/NP de 0,5/1. Pour ces deux ratios ON/NP (0,2/1 et 0,5/1) l'adsorption d'ON sur les nanoparticules de CTAB se situe au maximum à 10%. Il est important de noter pour un ratio ON/NP de 1/1 on obtient un pourcentage d'adsorption d'oligonucléotide de 80% avec les nanoparticules de chitosan 5 KDa. (figure 6).

### Exemple V: Etude de la protection des ODN adsorbés sur les NP.

### 1) Matériels et méthodes.

L'intégrité de l'ODN EF3008AS seul et associé aux NP Chitosan a été déterminée dans 10% de sérum de veau nouveau né décomplémenté. Les divers complexes sont incubés dans 120 µL de NaCl 150 mM avec 10% de sérum de veau nouveau-né décomplémenté. A différents temps d'incubation, des échantillons de 15 µL sont prélevés, mélangés à un même volume d'une solution de formamide/eau (4/1), 0.01% de Bleu de Bromophénol et 0.01% de Xylène Cyanol et congelés à -20°C. Les NP sont dégradées par du NaOH à pH=12 pendant 2 heures à 37°C. Les ODN sont alors récupérés par une double extraction au phénol/chloroforme/alcool isoamylique (25/24/1) et sont ensuite précipités dans 10 volumes d'acétone contenant 2% de perchlorate de lithium pendant 30 minutes à -20°C.

Les ODN sont ensuite repris dans 5 µL de solution formamide/eau (4/1), 0.01% de bleu de Bromophénol et 0.01% de Xylène Cyanol, chauffés 5 minutes à 95°C puis mis dans la glace. Ils sont analysés sur un gel dénaturant à 20% de polyacrylamide, 7M d'urée et TBE 1X. Le gel est scanné par un phosphoimager (Storm 840, Molecular Dynamics). La dégradation des ODN est quantifiée en effectuant le rapport du signal des bandes correspondant aux ODN intacts et aux ODN dégradés.

### 2) Résultats.

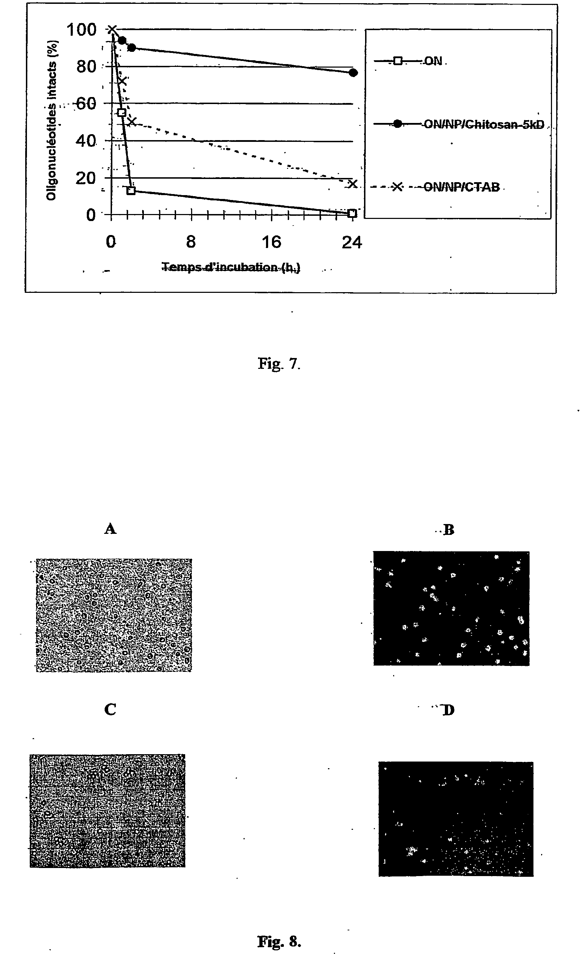

La figure 7 montre qu'il y a une bonne protection de l'ODN 30 mer par les NP-Chitosan.

### Exemple VI : Transfection de cellules humaines par des NP-Chitosan chargées en ODN.

### 1) Matériels et Méthodes.

### a) Préparation des cellules.

Des cellules humaines CEM4FX ont été utilisées. 500 000 cellules en suspension sont ensemencées dans des plaques 12 puits (TPP, Suisse) dans 850 µl d'un milieu RPMI contenant 10% de SVF (sérum de veau foetal)

### b) Transfection des cellules.

150 µl de la suspension de nanoparticules préparée précédemment sont mis au contact des cellules pendant 24 heures.

### c) Fixation des cellules.

Les cellules sont ensuite fixées avant de pouvoir effectuer une observation en fluorescence. Au bout des 24 heures d'incubation des cellules en présence des nanoparticules, 1 µg de propidium iodide est ajouté à chaque puits. Il permet de reconnaître les cellules mortes en les colorant en rouge.

Les cellules sont ensuite centrifugées à 1000 tr/min pendant 5 min. Le surnageant est prélevé et les cellules sont lavées une fois encore avec du PBS. 200 µl de PBS contenant 4% de formaldéhyde sont alors ajoutés et les cellules sont laissées à température ambiante pendant 30 minutes. Puis les cellules sont lavées deux fois avec du PBS 0.1X. Un autre lavage avec de l'eau stérile est également effectué pour terminer. 20 µL de poly-lysine sont ajoutés à la fin. L'observation des cellules se fait au microscope équipé d'un filtre à 480 nm.

### 2) Résultats.

Chacune des observations des figures 8, 9 et 10 a été effectuée pour les deux types d'oligonucléotides 7 bases et 11 bases.

Les cellules en présence de nanoparticules Chitosan 5 KDa-ODN sont très fluorescentes et aucune toxicité n'est visible. En présence de la formulation nanoparticules Chitosan 5 KDa-Cyd-ODN, elles sont également fluorescentes mais plusieurs cellules mortes sont visibles.

Les nanoparticules Chitosan 10 KDa-Cyd-ODN présentent également une bonne fluorescence mais aussi une toxicité légèrement plus importante que les nanoparticules 5KDa-Cyd-ODN. En présence de nanoparticules 30 KDa-Cyd-ODN, les cellules sont également très fluorescentes, mais le nombre de cellules mortes est encore plus important que dans les deux cas précédents.

Dans le cas des cellules incubées en présence de nanoparticules CTAB-Cyd-ON, la fluorescence est faible et la toxicité très importante. Le complexe CTAB-ODN ne montre aucune fluorescence cellulaire.

Dans le cas des cellules incubées en présence de Cytofectine-ODN aucune fluorescence cellulaire n'est observée.

### Exemple VII: Transfection in vitro des cellules NIH-3T3 EWS-Flil par des complexes plasmide-NP-Chitosan.

### 1) Matériels et Méthodes.

### a) Plasmide couplé à la rhodamine.

10 µg de plasmide β-Galactosidase sont marqués avec un fluorophore rouge Oregon Green 546 (ULYSIS^{®} Nucleic Acid Labeling Kits, Molecular Probes). Ce fluorophore donne une fluorescence rouge (Ex/Em est 555/570).

1 µg de plasmide marqué et 3 µg de plasmide non marqué sont mélangés à des NP-Chitosan dans 200 µL de NaCl à 150 mM, et vortexés puis incubés pendant 30 minutes à température ambiante. Les cellules NIH-3T3 *EWS-Fli*1 adhérentes sont ensemencées dans des plaques à 6 puits (TPP, Suisse) 24 heures avant la transfection afin d'atteindre 60-70% de confluence.

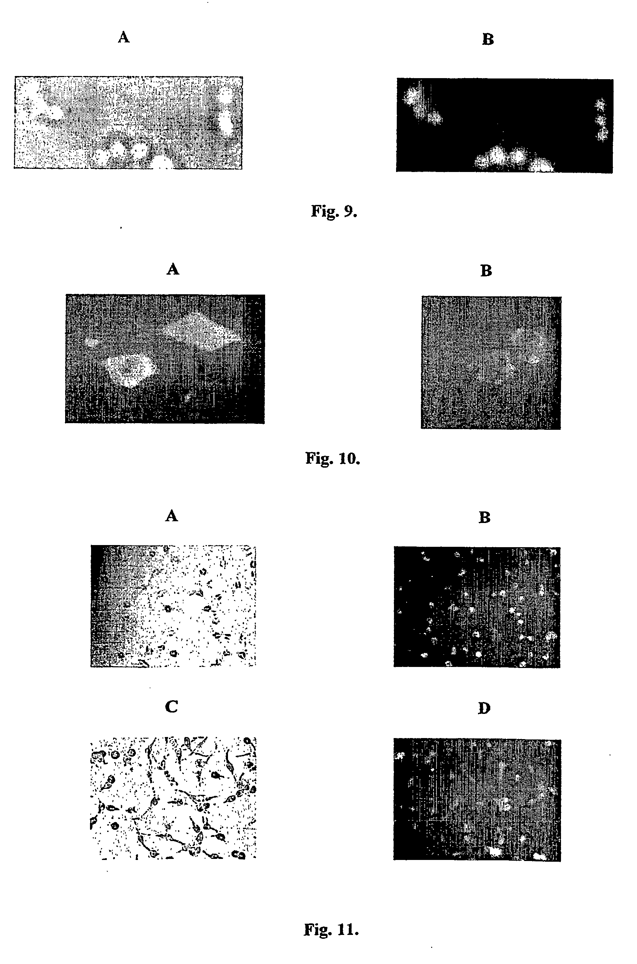

30 minutes avant la transfection, les cellules sont lavées avec 2mL de PBS 1X par puits puis 800 µL de DMEM contenant 2% de SVF sont ajoutés dans chaque puits. Les complexes (200 µL) sont mis au contact de cellules NIH-3T3 *EWS-Fli*1 pendant 16 heures à 37°C. Les images des cellules fluorescentes sont présentées dans la photographie (figure 11B et 11D).

### 2) Résultats.

On observe une fluorescence des cellules lorsqu'elles sont couplées aux nanoparticules de chitosan. (figures 11B et 11D).

### Exemple VIII :Induction de l'expression in vivo de la β-galactosidase par le plasmide pCMVβ-gal vectorisé par les NP-chitosan 5kDa.

### 1) Matériels et méthodes.

### a) Traitement des animaux.

Les cellules EWS-Fli1 sont obtenues par transfection des cellules NIH 3T3 avec l'oncogène EWS-Fli1. Elles sont ensuite mises en culture. Lorsque les cellules ont atteint 70% de confluence, elles sont mises en suspension dans du PBS à la concentration de 6,5X10⁶ cellules/ml. Ces cellules sont par la suite inoculées à des souris nude par voie sous cutanée à raison de 1,3x10⁶ cellules/souris. 7 jours après inoculation des cellules (la tumeur devient visible à l'oeil nu), les animaux sont traités soit avec 100 µl NP-chitosan 5kDa (60 µg) associé à 10 µg de plasmide pCMVβ-gal ou soit avec 100 µl de plasmide pCMVβ-gal (10 µg). 24 heures après le traitement, la tumeur a été prélevée pour une étude histologique après immersion dans du Glyo-Fixx (Shandon).

### b) Détection de l'expression de la GFP dans la tumeur par la technique d'immunohistochimie.

Les fragments de tumeur sont coulés dans de la paraffine et marqués par une solution de hemateine-eosine-saffranine. La protéine β-gal est détectée sur une coupe de fragment de la tumeur de 4 µm par un anticorps anti-β-gal (Clontech) (1 :30) et suivie par le Power Vision Histostaining kit (Immuno Vision Technology, CA) après le contre marquage, les échantillons sont observés au microscope (Zeiss) associé à une caméra CDD Camera Imaging System (Sony).

### 2) Résultats.

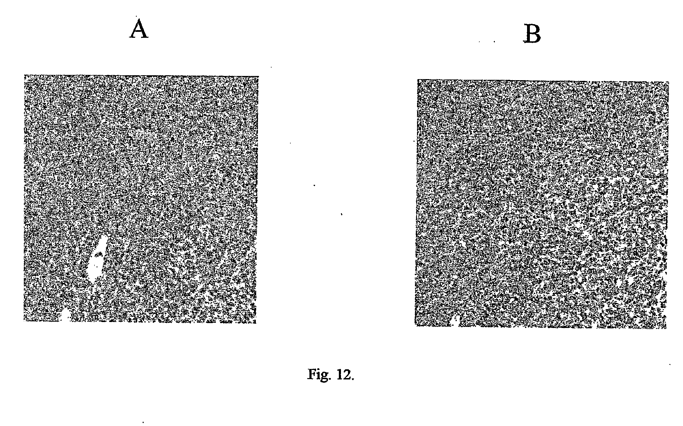

L'activité β-gal après 24 heures est observée seulement lorsque les animaux sont transfectés avec le plasmide pCMVβ-gal associé au NP-chitosan 5KDa (Figure 12 B) tandis lorsque les animaux sont transfectés avec le plasmide pCMVβ-gal seul on ne note pas d'activité β-gal (Figure 12 A).

### Exemple IX : Pénétration intracellulaire des ODN antisens vectorisés par les nanoparticules-Chitosan 5 et 10 kDa dans les cellules NIH 3T3 EWS/Fli-1

### 1) Matériels et méthodes.

Les cellules NIH 3T3 EWS/Fli-1 sont ensemencées 24 h avant leur transfection dans du milieu de culture sans puromycine dans des chambres de culture montées sur lamelle (labtek^{®} 4 puits, Nunc Inc.) à raison de 100 000 cellules par puits. Les cellules sont rincées au PBS et incubées pendant 24 h dans 400 µl de milieu OPT1-MEM1 supplémenté par 2,5% de SVF, 100 U/ml de pénicilline et 100 µg/ml de streptomycine en présence des différents complexes NP-chitosan :ODN-FITC aux ratios de masse 5 :1, 10 : 1 et 20 :1. Ces complexes sont préparés dans du NaCl 150 mM pou un volume final de 100 µl. Deux témoins ont été utilisés correspondant d'une part, aux cellules incubées avec l'ODN As-FITC libre (1 µg) et d'autre part, aux cellules incubées avec 1 nmole de fluorescéine (Aldrich). La pénétration des ODN AS-FITC dans les cellules est visualisée par microscopie confocale à l'aide d'un appareil Leica TCS NT (Leica) monté sur un microscope inversé Leica DM IRB/E.

### 2) Résultats.

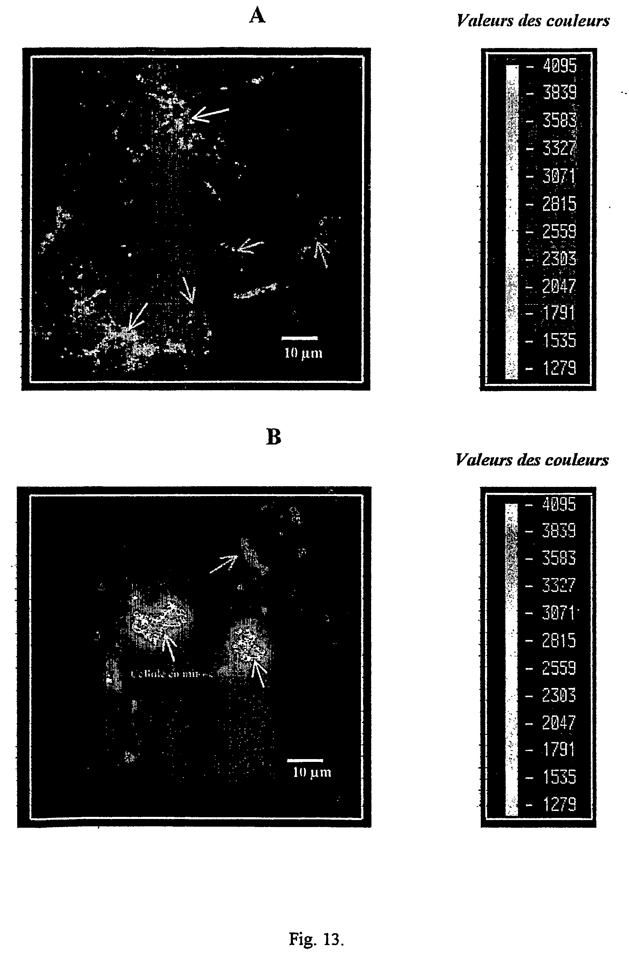

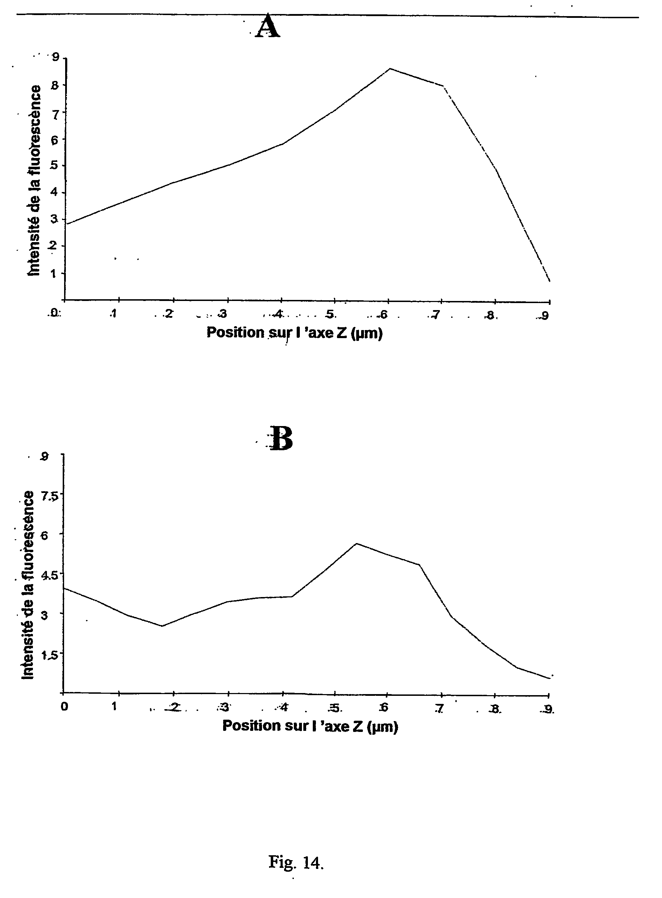

Aucune cellule fluorescente n'a été observée. Après leur incubation avec le FITC seul et l'ODN AS-FITC non vectorisé. Les images de la figure 13A et 13B présentent un champ des cellules incubées respectivement avec les complexes NP-chitosan 5kDa :ODN AS-FITC et NP-chitosan 10kDa : :ODN AS-FITC au ratio (5 :1). Elles montrent que la fluorescence est dispersée dans le cytosol quand les ODN-FITC sont vectorisés par les NP-chitosan 5 kDa et concentrée au niveau du noyau dans le cas des NP-chitosan 10 kDa. Les courbes de la figure 14A et 14B présentent la somme des intensités de fluorescence enregistrées dans différents plans Z de la cellule. Pour une profondeur de 0 à 1 µm, la fluorescence est située au niveau de la membrane apicale des cellules. Une profondeur de 8 à, 9 µm correspond à leur membrane basale. Entre 1 à 8 µm, la fluorescence est localisée au niveau intracellulaire. Ces courbes montrent que pour les 2 types de NP-chitosan au ratio NP-chitosan :ODN 5 :1, la fluorescence plus faible au niveau de la membrane basale des cellules que dans la membrane apicale exposée au milieu de culture contenant les ODN-FITC. Pour les 2 types de chitosan au ratio NP-chitosan :ODN (5 :1), la fluorescence est majoritairement localisée dans la fraction intracellulaire. Ces courbes confirment que les ODN AS pénètrent dans les cellules. L'intensité de fluorescence à l'intérieur des cellules est plus forte quand l'ODN-FITC est vectorisé par les NP-chitosan 5 kDa. La localisation cellulaire de la fluorescence est la même pour les ratios NP-chitosan :ODN (10 :1) et (20 :1) que le ratio (5 :1) pour les 2 types de NP-chitosan.

### Exemple X : Inhibition de la croissance tumorale après administration intratumorale des NP-chitosan à des souris nude qui ont développé la forme sous-cutanée du sarcome d'Ewing.

### 1) Matériels et Méthodes.

35 souris *nude* femelles âgées de 6-8 semaines sont irradiées à 5 Grey. 24 heures après l'irradiation, les souris reçoivent par voie sous-cutanée dans le flanc droit 200 µl d'une suspension de cellules NIH 3T3 EWS-Fli à 5x10⁶ cellules/ml dans du PBS. Les animaux développent une tumeur au bout de 14-21 jours. 4 groupes de 7 animaux sont constitués (groupe PBS, groupe ODN EF3008AS, groupe NP-chitosan 5 kDa ODN EF3008AS et groupe NP-chitosan 5kDa ODN contrôle. Les groupes sont randomisés et le traitement débutera lorsque la tumeur aura atteint 2 mm³. Le traitement consiste en 5 administrations intratumorales des produits par intervalle de deux jours. L'efficacité des traitements sera évaluée par la mesure de la croissance tumorale des animaux traités par rapport aux témoins. La croissance tumorale sera évaluée par le volume tumoral (VT). VT sera calculé comme suit : VT(mm3)= a²xb/2 où a et b représentent respectivement le plus petit et le plus grand diamètre de la tumeur. L'analyse statistique des résultats est réalisée à l'aide du logiciel informatique StatView^{®} (Abacus Concepts, Berkeley, USA).

### 2) Résultats.

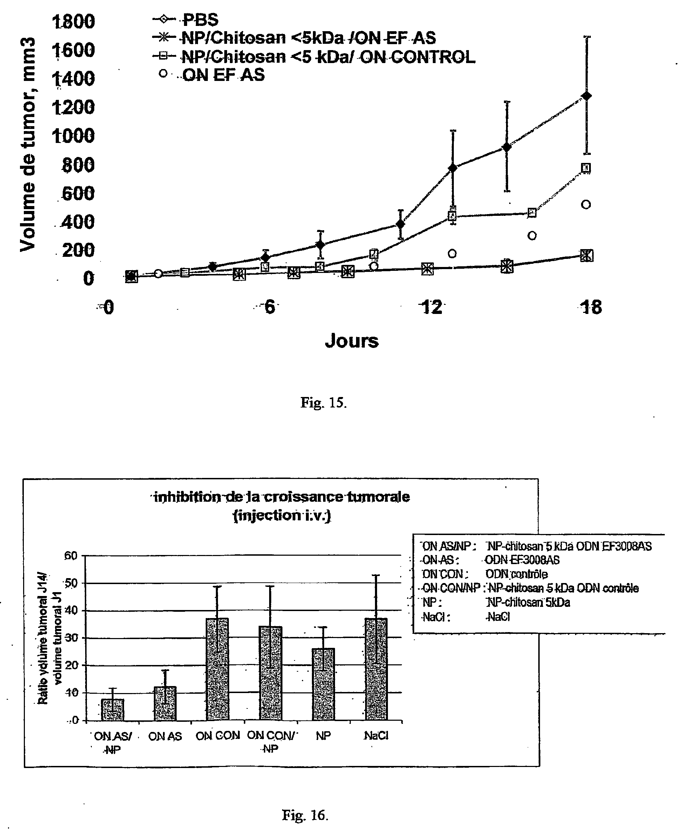

Les résultats d'inhibition de croissance tumorale (figure 15) montrent une inhibition de la croissance lorsque les animaux sont traités avec de l'ODN EF3008AS et les NP-chitosan 5kDa ODN EF3008AS. Cependant on note une inhibition de la croissance plus importante lorsque les animaux sont traités avec les NP-chitosan 5kDa ODN EF3008AS Par rapport aux animaux qui ont reçu de l'ODN EF3008AS. Les animaux témoins qui ont reçu du PBS ou des NP-chitosan 5kDa ODN control montrent une faible inhibition de la croissance tumorale.

### Exemple XI : Inhibition de la croissance tumorale après administration intraveineuse du traitement à des souris qui ont développé la forme sous-cutanée du sarcome d'Ewing

### 1) Matériels et méthodes.

36 souris nude femelles âgées de 6-8 semaines sont irradiées à 5 grey. 24 heures après l'irradiation, les souris reçoivent par voie sous-cutanée dans le flanc droit 200µl d'une suspension de cellules EWS-Flil à 5X10⁶ cellules/ml dans du PBS. Les animaux développent une tumeur au bout de 10-20 jours. 6 groupes de 6 animaux sont constitués (groupe NP-chitosan 5kDa ODN EF3008AS(ON AS/NP), ODN EF3008AS (ON AS), ODN contrôle (ON CON), NP-chitosan 5kDa ODN contrôle (ON CON/NP), NP-chitosan 5kDa (NP) et NaCl à 0,9% (NaCl)). Les groupes sont randomisés et le traitement débutera lorsque la tumeur aura atteint 10-40 mm³. Le traitement consiste en 5 administrations intra-veineuses des produits par intervalle de 2 ou 3 jours. L'efficacité des traitements sera évaluée au bout de 15 jours par la moyenne des ratios entre la mesure du volume tumoral à J14 et la mesure du volume tumoral à J1 des groupes d'animaux traités par rapport aux groupes témoins. Le volume tumoral est calculé comme dans l'exemple X.

### 2) Résultats.

Les résultats de la croissance tumorale (Figure 16) montrent une inhibition de la croissance lorsque les animaux sont traités avec de l'ODN EF3008AS (groupe ON AS) et les NP-chitosan 5kDa ODN EF3008AS (ON AS/NP). Cependant, on note une inhibition de la croissance tumorale plus importante lorsque les animaux sont traités avec les NP-chitosan 5kDa ODN EF3008AS (groupe ON AS/NP) par rapport aux animaux qui ont reçu de l'ODN EF3008AS sans NP-chitosan (groupe ON AS). Les animaux des groupes contrôles qui ont reçu du NaCl, des NP-chitosan seules (groupe NP) ou de l'ODN contrôle avec ou sans NP-chitosan (groupes ON CON/NP et ON CON respectivement) montrent une faible inhibition de la croissance tumorale.

### Exemple XII : Inhibition de l'expression de la protéine GFP et inhibition de l'expression de l'ARNm de la protéine GFP in vivo par l'ARNsi vectorisé par les nanoparticules.

### 1) Matériels et Méthodes.

Les cellules adhérentes HeLa transfectées avec la GFP (HeLa-GFP) en culture en monocouche sont prélevées lorsqu'elles ont atteint 70% de confluence et remises en suspension dans du PBS à raison de 1X10⁷ cellules/ml. Les cellules sont ensuite administrées par voie sous-cutanée dans le flanc droit à des souris nude à raison de 2X10⁶ cellules/souris. 7 jours après inoculation des cellules, les animaux sont repartis en différents groupes et traités par administration intra tumorale de différentes préparations de la façon suivante :
Groupe 1 (100 µl d'une préparation contenant 10 µg d'ON GFP double brin cible de l'ARNsi), Groupe 2 (100 µl de l'ARNsi contrôle seul), Groupe 3 (100 µl de l'ARNsi contrôle vectorisé avec 10 µg de NP-chitosan 5kDa), Groupe 4 (100 µl d'ON antisense GFP seul) Groupe 5 (100 µl d'ON antisense GFP vectorisé par les NP-chitosan 5 KDa). 24 heures après le traitement, les tumeurs sont prélevées et divisées en deux parties. La partie destinée à l'étude histologique est immergée dans une solution de GlyoFixx (Shandon) et la partie utilisée pour le Northern blot est conservée dans l'azote liquide.

### a) Détection de l'expression de la GFP dans la tumeur par la technique d'immunohistochimie.

Les fragments tumoraux sont coulés dans la paraffine et marqués par une solution d'hemateine-eosine-saffarine. La GFP est détectée sur une coupe de 4 µm par un anti-corps anti-GFP (Clontech) (1 :30) et suivie par le Power Vision Histostaining kit (ImmunoVision Technology CA) après le contre marquage, les échantillons sont observés au microscope (Zeiss) associé à une caméra CDD Camera Imaging System (Sony).

### b) Détection de l'expression de l'ARNm de la GFP dans la tumeur par la techniques de Northern Blot.

Les fragments tumoraux ont été broyés dans une solution contenant 400 µl de guanidium thiocyanate 4M, 25 mM de citrate Na (pH 7), 0,5% de sarcosyl et 0,1M de β-mercaptoethanol à 0°C. Après homogénéisation, l'ARN a été extraite dans le phenol comme suit : 40 µl de solution d'acetate de Na 2M (pH4) sont ajoutés à 400 µl d'H₂O saturé de phenol et 120 µl de chloroforme :alcool isoamylique (49 :1). Le mélange est centrifugé à 1300 rpm pendant 15 minutes. 300 µl de la phase aqueuse est précipité avec 300 µl d'isopropanol à -20°C. Le culot ainsi obtenu est lavé avec de l'éthanol à 70%. Après séchage le culot est repris avec 10 µl d'H₂O distillée. L'électrophorèse de 2 µg d'ARN total a été réalisée sur un gel d'agarose 1% dans du tampon MOPS contenant 6,3% de formaline. L'ARN a été transféré sur une membrane de nitrocellulose (BA-S85, Schleicher&Shuell) dans un tampon SSC (10X). L'ARNm de la GFP est détecté par la technique de Northern blot avec un fragment d'ADNc de la GFP de 770pb marqué par α³²-P dCTP 300 ci/mM (ICN, France) et Prime-a-gene labeling system (Promega). Après lavage, la membrane de nitrocellulose a été analysée par le Storm 840 phosphorimager (Molecular Dynamic).

### 2) Résultats.

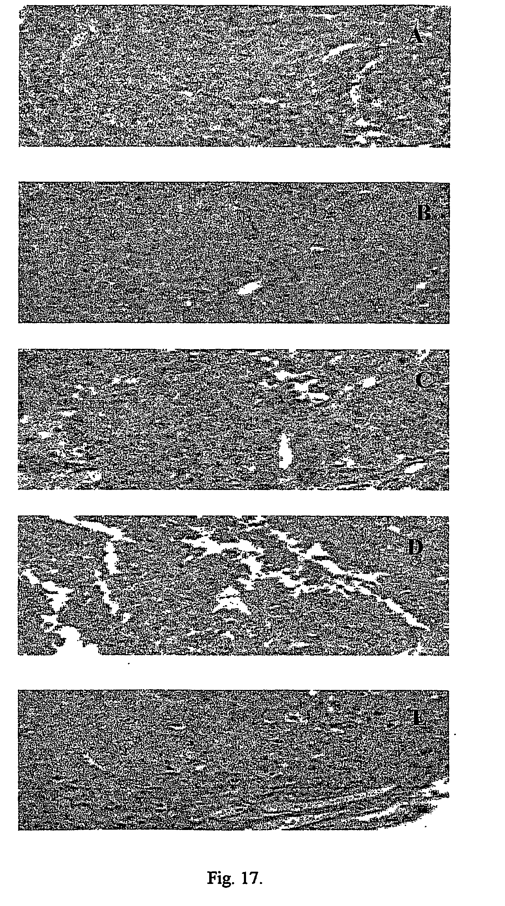

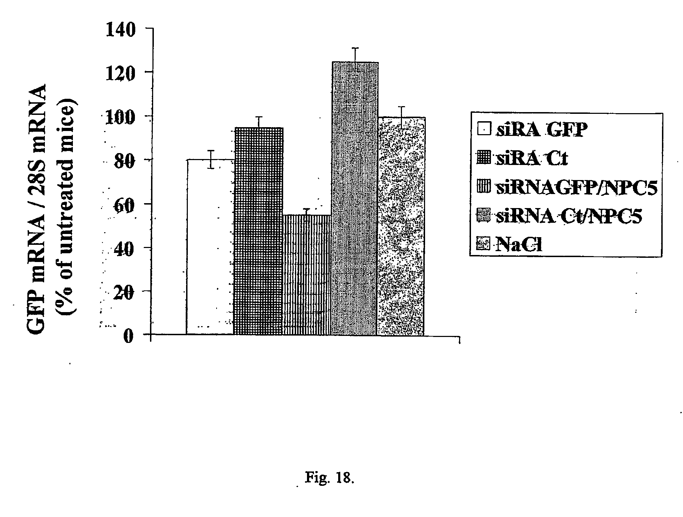

L'inhibition de la synthèse de la GFP est observé seulement lorsque l'ARNsi est vectorisé avec les NP-chitosan 5kDa comme le montre la figure 17 (D). Dans tous les autres cas on ne note pas d'inhibition de la GFP (figure 17 A, B, C et E). L'activité antisens de l'ARNsi vectorisé par les NP-chitosan 5kDa est confirmé par la technique de Northern blot. On note une inhibition de la synthèse d'ARNm de la GFP de 50% au bout de 24h lorsque l'ARNsi est vectorisé par les nanoparticules (figure 18). Ces résultats démontrent que l'ARNsi vectorisé par les nanoparticules interfère avec le gène d'expression de la GFP *in vivo.*

### RÉFÉRENCES BIBLIOGRAPHIQUES

Balassa, L.L., and Grove, B., 1972. U.S. Pat. 3, 632, 754.
Behr, J.P., Demeneix, B., Loeffler, J.-P., Perez-Mutul, J., 1989. Efficient gene transfer into mammalian primary end-crine cells with lipopolyamine-coated DNA. Proc. Natl. Acad. Sci. USA 86, 6982-6986.
Boussif, O., Lezoualch, F., Zanta, M.A., Mergny, M.D., Scherman, D., Demeneix, B., Behr, J.P., 1995. A versatile vector for gene and oligonucleotide delivery into cells in culture and in vivo: polyethylenimine. Proc. Natl. Acad. Sci. USA 92 , 7297-7310.
Chandy, T., and Sharma C.P., 1990. Biomat. Art. Cells. Art. Org., 18, 1.
Chavany, C., Le Doan T., Couvreur, P., Puisieux, F., Helene, C., 1992. Polyalkylcyanoacrylate nanoparticules as polymeric carriers for antisense oligonucleotides, Pharm. Res. 9, 441-449.
Crook, S.T., 1995. Delivery of oligonucleotides and polynucleotides. J. Drug Targeting 3, 185-190.
Douglas, J.T., Curiel, D.T., 1995. Targeted gene therapy. Tumor Targeting 1, 67-84.
Erbacher, P., Zou, S., Bettinger, T., Steffan, A.M., Remy, J.S., 1998, Chitosan-Based Vector/DNA Complexes for Gene Delivery: Biophysical Characteristics and Transfection Ability. Pharm. Res. 15, 1332-1339.
Felgner, P.L., 1990. Particulate systems and polymers for in vitro and in vivo delivery of polynucleotides. Adv. Drug Deliv. Rev. 5, 163-187.
Fernandez-Urrusuno, R., Calvo P., Remunan-Lopez, C., Vila-Jato, J.L., and Alonso, M.J. 1999. Enhancement nasal absorption of Insulin Using Chitosan., Pharm. Res., 16, 1576-1581.
Fradet, G., Brister, S., Mulder, D.S., Lough, J., and Averbach, B.L., 1986. In chitin in nature and Technology (R. Muzzarelli, C. Jeuniaux, and G.W. Gooday, eds?), Plenum Press, New York, p. 443.
Fynan, E.F., Webster, R.G., Fuller, D.H., Haynes, J.R., Santoro, J.C., Robinson, H.L., 1993. DNA vaccines: protection immunization by parenteral, mucosal, and gene-gun inoculations. Proc. Natl. Acad. Sci. USA 90, 11478-11482.
Hillyard, I.W., Doczi, J., and Kiernan, P.B., 1964. Proc. Soc. Exp. Biol. Med., 115, 1108.
Hirano, S., Seino, H., Akiyama, Y., and Nonaka, I., 1990. in Progress in Biomedical polymers (C.G. Gebelein and R.L. Dunn, eds), Plénum Press, New York, p.283.
Kabanov, A.V., Alakhov, V.Y., 1993. Micelles and amphiphilic block copolymers as vehicles for drug delivery. In: Alexandris, P., Lindman, B. (Eds.), Amphiphilic block copolymers: Self Assembly and Applications. Elsevier, Amsterdam, pp. 1-30.
Knapczyk, J., Krowczynski, L., Pawlik, B., and Liber Z., 1984. In chitin and Chitosan: Sources, Chemistry, Biochemistry, Physical Properties and Applications (G. Skjak-Barek, T. Anthonsen and Sandford P., eds., Elsevier Applied Science, London, p. 665.
Kobayashi, T., Otsuka, S., and Yugari, Y., 1979. Nutr. Rep. Int., 20, 677.
Kreuter, J. Colloidal Drug Delivery Systems, Marcel Decker, New York, 1994, 219-342.
Lasic, D., Templeton, N., 1996. Liposomes in gène therapy. Adv. Drug Deliv. Rev. 20, 221-266.
Ledley, F.D., 1994. Non-viral gene delivery. Curr. Opin. Biotechnol. 5, 626-636.
Morgan, R.A., Anderson, W.F., 1993. Human gene therapy. Annu. Rev. Biochem. 62, 191-217.
Mumper, R.J., Duguid, J.G., Anwer, K., Barron, M.K., Nitta, H., Rolland, A.P., 1996. polyvinyl derivatives as novel interactive polymers for controlled gene delivery to muscle. Pharm. Res. 13, 701-709.
Calvo, P., Remunan-Lopez, C., Vila-Jato, J.L., Alonso, M.J., 1997. Chitosan and chitosan/ethylene oxide-propylene oxide block copolymer nanoparticles as novel carriers for proteins and vaccines. Pharm. Res. 14, 1431-1436.
Calvo P., Boughaba, A.S., Appel, M., Fattal, E., Alonso, M.J., Couvreur, P., 1998. Oligonucleotide-chitosan nanoparticles as new gene therapy vector. Proceed. 2nd World Meeting APGI/APV, Paris, pp. 1111-1112.
Remy, J.S., Abdallah, B., Zanta, M.A., Boussif, O., Behr, J.-P., Demeneix, B., 1998. Gene transfer with lipospermines and polyethylenimines. Adv. Drug Deliv. Rev. 30, 85-95.
Rha, C.K., Rodriguez-Sanchez, D., and Kienzle-Sterzer, 1984. In Biotechnology of Marine Polysaccharides (R.R. Colwell, E.R. Pariser, A.J. Sinskey, eds.), Hemisphere Plulishing, Washington, p. 284.
Richardson, C.W., Kolbe, H.V.J., Duncan, R., 1999. Potential of low molecular mass Chitosan as a DNA delivery system: biocompatibility, body distribution and ability to complex and protect DNA. Int. J. Pharm., 178, 231-243.
Roberts, G.A.F., 1992. In chitin Chemistry (G.A.F. Roberts ed.), Mac Millan Press, Hounmills, p. 274.
Roberts, G.A.F., in chitin chemistry 1992, (G.A.F. Roberts ed.), Mac Milan Press, Houndmills, p.1.
Smith, R.L., 1984. U.S. Pat. 4, 474, 769.
Struszczyk, H., Wawro, D., and Niekraszewicz, A., 1991. In Advances in chitin and Chitosan (C.J. Brine, P.A. Sandford, and J.P. Zikakis, eds.), Elsevier Applied Science, London, p.580.
Tang, M.X., Redmann, C.T., Szoka, F.C., 1996. In vitro gene delivery by degraded polyamidoamine dendrimers. Bioconjugate Chem. 7, 703-714.
Verma, I.M., Somia, N., 1997. Gene therapy-promises, problems and prospects. Nat. Med. 389, 239-242.
Wilson, J.A., Ping, A.J., Krauss, J.C., Mayo-Bond, L., Rogers, C.E., Anderson, D.C., Tod III, R.F., 1990. Correction of CD18 deficient lymphocytes by retrovirus mediated gene transfer. Science, 248, 1413-1416.
WU, G., Wu, C., 1987. Receptor-mediated in vitro gene transformation by soluble DANN carrier system. J. Biol. Chem. 262, 4429-4432.
Zelphati, O., Szoka, F.C. Jr., 1996. Liposomes as carriers for intracellular delivery of antisense oligonucleotide : real or magic bullet ? J. Control. Release 41, 99-119.
Zobel, H.P., Kreuter, J., Werner, D., Noe, C.R., Kumel, G., Zimmer, A., 1997. Cationic polyhexylcyanoacrylate nanoparticles as carriers for antisense oligonucleotide, Antisense Nucleic Acid Drug Dev. 7, 483-493.
Yang, S.C., Ge, H.X., Hu, Y., Jiang, X.Q., Yang, C.Z., 2000. Formation of positively charged poly(butyl cyanoacrylate) nanoparticles stabilized with chitosan, Colloid Polym. Sci.278, 285-292.
Couvreur, P., Kante, B., Roland, M., Guiot, P., Baudin, P., Speiser, P., 1979. Polycyanoacrylate nanocapsules as potential lysosomotropic carriers: preparation, morphological and sorptive properties. J. Pharm. Pharmacol., 31, 331-332.

## Revendications

1. Système de vectorisation et de délivrance de substances d'intérêt-thérapeutique ou de diagnostic, du type constitué de nanoparticules, **caractérisé en ce que** lesdites nanoparticules présentent une distribution de taille homogène et comprennent (i) au moins un polymère et (ii) au moins un polysaccharide chargé positivement non toxique.

2. Système de vectorisation et de délivrance de substances d'intérêt selon la revendication 1, **caractérisé en ce que** lesdites substances sont internalisées ou adsorbées au niveau desdites nanoparticules.

3. Système de vectorisation et de délivrance de substances d'intérêt selon l'une des revendications 1 ou 2, **caractérisé en ce que** les substances d'intérêt sont anioniques ou comprennent une région anionique.

4. Système de vectorisation et de délivrance de substances d'intérêt selon l'une des revendications 1 à 3, **caractérisé en ce que** le polymère est un poly(cyanoacrylate d'alkyle) dans lequel le groupe alkyle, linéaire ou ramifié, comprend de 1 à 12 atomes de carbone.

5. Système de vectorisation et de délivrance de substances d'intérêt selon l'une des revendications 1 à 4, **caractérisé en ce que** le polysaccharide chargé positivement est le chitosan ou l'un de ses dérivés.

6. Système de vectorisation et de délivrance de substances d'intérêt selon l'une des revendications 1 à 5, **caractérisé en ce que** les polysaccharides chargé positivement, tout particulièrement le chiton ou un de ses dérivés, a une masse moléculaire inférieure à 100 000 Da, de préférence inférieur 30 000 Da.

7. Système de vectorisation et de délivrance de substances d'intérêt selon l'une des revendications 1 à 6, **caractérisé en ce que** les nanoparticules comprennent au moins un composé apte à complexer les substances d'intérêt.

8. Système de vectorisation et de délivrance de substances d'intérêt selon la revendication 7, **caractérisé en ce que** le composé apte à complexer les substances d'intérêt est un oligosaccharide cyclique, de préférence une cyclodextrine neutre ou chargée, native, branchée ou polymérisée ou modifiées chimiquement.

9. Système de vectorisation et de délivrance de substances d'intérêt selon l'une quelconque des revendications précédentes, caractérisé en que les substances d'intérêt sont choisies dans le groupe comprenant l'ADN ou des oligodésoxyribonucléotides ou des oligoribonucléotides notamment des antisens ou des ARN interféron (ARNsi), ou encore des peptides, polypeptides ou protéines, des composés chimiques.

10. Système de vectorisation et de délivrance de substances d'intérêt selon l'une quelconque des revendications précédentes, caractérisé en que les nanoparticules présentent un diamètre homogène compris entre 10 et 300 nm.

11. Procédé de préparation de nanoparticules entrant dans la composition d'un système de vectorisation et de délivrance de substances d'intérêt selon l'une quelconque des revendications précédentes, caractérisé en qu'il comprend la polymérisation à pH acide supérieur à 1 des monomères en présence du polysaccharide chargé positivement pour obtenir une suspension de nanoparticules.

12. Procédé selon la revendication 11, **caractérisé en ce que** le polysaccharide est ajouté en début de polymérisation.

13. Procédé selon l'une des revendications 11 ou 12, **caractérisé en ce que** le pH acide est supérieur à 3.

14. Procédé selon l'une des revendications 11 à 13, **caractérisé en ce que** la polymérisation est réalisée en présence d'un composé ralentisseur de polymérisation comme SO₂ ou l'hydroquinone, qui se fixe sur les groupes OH du milieu aqueux de polymérisation ou du polysaccharide.

15. Procédé selon l'une des revendications 11 à 14, **caractérisé en ce que** la quantité de polysaccharide est avantageusement comprise entre 0,01% et 5% et de préférence entre 0,1 et 1 % par rapport au poids de la suspension.

16. Procédé selon l'une des revendications 11 à 15, **caractérisé en ce qu'**il comprend l'addition polyisohexylcyanoacrylate contenant du S02 dans une solution d'acide citrique à un pH compris entre 1 et 5 et contenant un tensioactif non ionique, éventuellement de la cyclodextrine et du chitosan de faible poids moléculaire (5000 ou 10000 Da).

17. Procédé selon l'une des revendications 11 à 16, **caractérisé en ce qu'**il comprend l'addition des substances d'intérêt en même temps que le polysaccharide.

18. Procédé selon l'une des revendications 11 à 16, **caractérisé en ce qu'**il comprend l'addition des substances d'intérêt après la formation des nanoparticules.

19. Une suspension de nanoparticules susceptible d'être obtenue par un procédé selon l'une quelconque des revendications 11 à 18, **caractérisée en ce qu'**elle comprend de 0,01% à 5%, en particulier entre 0,1% et 1% de polysaccharide chargé positivement par rapport au poids de la suspension.

20. Une suspension de nanoparticules selon la revendication 19, **caractérisée en ce qu'**elle comprend de 0,5% à 50% de substances d'intérêt par rapport au poids de la suspension.

## Claims

1. A vectorisation and delivery system for substances of therapeutic or diagnostic interest, of the type formed by nanoparticles, **characterised in that** said nanoparticles exhibit distribution of homogeneous size and include (i) at least one polymer and (ii) at least one positively charged, non-toxic polysaccharide.

2. The vectorisation and delivery system for substances of interest according to Claim 1, **characterised in that** said substances are internalised or adsorbed on said nanoparticles.

3. The vectorisation and delivery system for substances of interest according to either of Claims 1 or 2, **characterised in that** the substances of interest are anionic or include an anionic region.

4. The vectorisation and delivery system for substances of interest according to any of Claims 1 to 3, **characterised in that** the polymer is an alkyl poly(cyanoacrylate) in which the alkyl linear or branched group includes 1 to 12 carbon atoms.

5. The vectorisation and delivery system for substances of interest according to any of Claims 1 to 4, **characterised in that** the positively charged polysaccharide is chitosan or one of its derivatives.

6. The vectorisation and delivery system for substances of interest according to any of Claims 1 to 5, **characterised in that** the positively charged polysaccharides, in particular chiton or one of its derivatives, has a molecular mass of lower than 100,000 Da, preferably lower than 30,000 Da.

7. The vectorisation and delivery system for substances of interest according to any of Claims 1 to 6, **characterised in that** the nanoparticles include at least one compound capable of complexing the substances of interest.

8. The vectorisation and delivery system for substances of interest according to Claim 7, **characterised in that** the compound capable of complexing the substances of interest is a cyclic oligosaccharide, preferably a neutral or charged, native, branched or polymerised or chemically modified cyclodextrin.

9. The vectorisation and delivery system for substances of interest according to any of the preceding claims, **characterised in that** the substances of interest are chosen from the group comprising DNA or oligodeoxyribonucleotides or oligoribonucleotides, in particular antisenses or interferon RNAs (RNAis), or else peptides, polypeptides or proteins, of the chemical compounds.

10. The vectorisation and delivery system for substances of interest according to any of the preceding claims, **characterised in that** the nanoparticles exhibit a homogeneous diameter between 10 and 300 nm.

11. A method for preparing nanoparticles entering into the composition of a vectorsation and delivery system for substances of interest according to any of the preceding claims, **characterised in that** it includes polymerisation at acid pH higher than 1 of the monomers in the presence of the positively charged polysaccharide in order to obtain a nanoparticle suspension.

12. The method according to Claim 11, **characterised in that** the polysaccharide is added at the start of polymerisation.

13. The method according to either of Claims 11 or 12, **characterised in that** the acid pH is higher than 3.

14. The method according to any of Claims 11 to 13, **characterised in that** the polymerisation is implemented in the presence of a polymerisation slowing compound such as SO₂ or hydroquinone, which fixes onto the OH groups of the polymerisation aqueous medium or the polysaccharide.

15. The method according to any of Claims 11 to 14, **characterised in that** the quantity of polysaccharide is advantageously between 0.01% and 5%, and preferably between 0.1 and 1% in relation to the weight of the suspension.

16. The method according to any of Claims 11 to 15, **characterised in that** it includes the addition of polyisohexylcyanoacrylate containing SO2 in a citric acid solution with a pH of between 1 and 5 and containing a non-ionic tensioactive, possibly cyclodextrin and chitosan with a low molecular weight (5,000 or 10,000 Da).

17. The method according to any of Claims 11 to 16, **characterised in that** it includes the addition of the substances of interest at the same time as the polysaccharide.

18. The method according to any of Claims 11 to 16, **characterised in that** it includes the addition of the substances of interest after the formation of the nanoparticles.

19. A nanoparticle suspension capable of being obtained by a method according to any of Claims 11 to 18, **characterised in that** it includes 0.01% to 5%, in particular between 0.1% and 1% positively charged polysaccharide in relation to the weight of the suspension.

20. A nanoparticle suspension according to Claim 19, **characterised in that** it includes 0.5% to 50% substances of interest in relation to the weight of the suspension.

## Patentansprüche

1. System zum Vektorisieren und Abgeben von Substanzen von therapeutischem oder diagnostischem Interesse des aus Nanopartikeln gebildeten Typs, **dadurch gekennzeichnet, dass** die Nanopartikel eine homogene Größenverteilung haben und (i) mindestens ein Polymer und (ii) mindestens ein nicht toxisches positiv geladenes Polysaccharid aufweisen.

2. System zum Vektorisieren und Abgeben von Substanzen von Interesse gemäß Anspruch 1, **dadurch gekennzeichnet, dass** die Substanzen bezüglich der Nanopartikel internalisiert und adsorbiert sind.

3. System zum Vektorisieren und Abgeben von Substanzen von Interesse gemäß seinem der Ansprüche 1 oder 2, **dadurch gekennzeichnet, dass** die Substanzen von Interesse anionisch sind oder einen anionischen Bereich aufweisen.

4. System zum Vektorisieren und Abgeben von Substanzen von Interesse gemäß einem der Ansprüche 1 bis 3, **dadurch gekennzeichnet, dass** das Polymer ein Poly(alkylcyanacrylat) ist, wobei die lineare oder verzweigte Alkylgruppe 1 bis 12 Kohlenstoffatome aufweist.

5. System zum Vektorisieren und Abgeben von Substanzen von Interesse gemäß einem der Ansprüche 1 bis 4, **dadurch gekennzeichnet, dass** das positiv geladene Polysaccharid Chitosan oder eines seiner Derivate ist.

6. System zum Vektorisieren und Abgeben von Substanzen von Interesse gemäß einem der Ansprüche 1 bis 5, **dadurch gekennzeichnet, dass** die positiv geladenen Polysaccharide, insbesondere das Chitosan oder eines seiner Derivate eine Molekülmasse von kleiner als 100 000 Da, vorzugsweise vorn kleiner als 30 000 Da aufweisen.

7. System zum Vektorisieren und Abgeben von Substanze von Interesse gemäß einem der Ansprüche 1 bis 6, **dadurch gekennzeichnet, dass** die Nanopartikel mindestens eine Verbindung aufweisen, die in der Lage ist, die Substanzen von Interesse zu komplexieren.

8. System zum Vektorisieren und Abgeben von Substanzen von Interesse gemäß Anspruch 7, **dadurch gekennzeichnet, dass** die Verbindung, die in der Lage ist, die Substanzen von Interesse zu komplexieren, ein zyklisches Oligosaccharid, vorzugsweise ein neutrales oder geladenes, natives, verzweigtes oder polymerisiertes oder chemisch modifiziertes Cyclodextrin ist.

9. System zum Vektorisieren und Abgeben von Substanzen von Interesse gemäß einem der vorhergehenden Ansprüche, **dadurch gekennzeichnet, dass** die Substanzen von Interesse, aus der Gruppe ausgewählt sind, die DNA oder Oligodesoxyribonukleotide oder Oligoribonukleotide, insbesondere Antisense oder RNA-Interferon (ARNsi), oder auch Peptide, Polypeptide oder Proteine und chemische Verbindungen aufweist.

10. System zur Vektorisieren und Abgeben von Substanzen von Interesse gemäß einem der vorhergehenden Ansprüche, **dadurch gekennzeichnet, dass** die Nanopartikel einen homogenen Durchmesser zwischen 10 und 300 nm besitzen.

11. Verfahren zum Herstellen von Nanopartikeln, die in die Zusammensetzung eines Systems zum Vektorisieren und Abgeben von Substanzen von Interesse gemäß einem der vorhergehenden Ansprüche eindringen, **dadurch gekennzeichnet, dass** es das polymerisieren der Monomere bei einem sauren pH-Wert von größer als 1 in Anwesenheit des positiv geladenen Polysaccharids aufweist, um eine Nanopartikelsuspension zu erhalten.

12. Verfahren gemäß Anspruch 11, **dadurch gekennzeichnet, dass** das Polysaccharid zu Beginn der Polymerisation hinzugefügt wird.

13. Verfahren gemäß einem der Ansprüche 11 oder 12, **dadurch gekennzeichnet, dass** der saure pH-Wert größer als 3 ist.

14. Verfahren gemäß einem der Ansprüche 11 bis 13, **dadurch gekennzeichnet, dass** die Polymerisation in Anwesenheit einer Polymerisationsverzögerungsverbindung, zum Beispiel SO₂ oder Hydrochinon erfolgt, die an die OH-Gruppen des wässrigen Polymerisationsmediums oder des Polysaccharids bindet.

15. Verfahren gemäß einem der Ansprüche 11 bis 14, **dadurch gekennzeichnet, dass** die Menge an Polysaccharid bezogen auf das Gewicht der suspension vorzugsweise zwischen 0,01% und 5% und vorzugsweise zwischen 0,1% und 1% liegt.

16. Verfahren gemäß einem der Ansprüche 11 bis 15, **dadurch gekennzeichnet, dass** es das Hinzufügen von SO2 enthaltendem Polyisohexylcyanacrylat in eine Zitronensäurelösung mit einem pH-Wert zwischen 1 und 5 aufweist, die ferner eine nicht ionische oberflächenaktive Substanz, möglicherweise Cyclodextrin und Chitosan mit einem geringen Molekulargewicht (5000 oder 10000 Da) enthält.

17. Verfahren gemäß einem der Ansprüche 11 bis 16, dadurch gekenntzeichnet, dass es das Hinzufügen der Substanzen von Interesse gleichzeitig mit dem Polysaccharid aufweist.

18. Verfahren gemäß einem der Ansprüche 11 bis 16, **dadurch gekennzeichnet, dass** es das Hinzufügen der Substanzen von Interesse nach dem Bilden der Nanopartikel aufweist.

19. Eine Nanopartikelsuspension, die geeignet ist, mittels eines Verfahrens gemäß einem der Ansprüche 11 bis 18 erhalten zu werden, **dadurch gekennzeichnet, dass** sie bezogen auf das Gewicht der Suspension 0,01% bis 5%, insbesondere zwischen 0,1% und 1% positiv geladenes Polysaccharid aufweist.

20. Eine Nanopartikelsuspension gemäß Anspruch 19, **dadurch gekennzeichnet, dass** sie bezogen auf das Gewicht der Suspension 0,5% bis 50% an Substanzen von Interesse aufweist.
